# EUROPÄISCHE PATENTSCHRIFT

(11) **EP 3 044 584 B1**
(45) Veröffentlichungstag und Bekanntmachung des Hinweises auf die Patenterteilung: **14.11.2018**
(21) Anmeldenummer: 14781452.9
(22) Anmeldetag: 09.09.2014
(51) Int. Cl.: G01N 33/14

(54) **VORRICHTUNG ZUR ERMITTLUNG EINER EIGENSCHAFT EINES FLÜSSIGEN MEDIUMS**
DEVICE FOR DETERMINING A CHARACTERISTIC OF A LIQUID MEDIUM
DISPOSITIF PERMETTANT DE DÉTERMINER UNE PROPRIÉTÉ D'UNE SUBSTANCE LIQUIDE

(30) Priorität: 15.09.2013 DE 102013015148
(43) Veröffentlichungstag der Anmeldung: 20.07.2016
(73) Patentinhaber: Steinfurth Mess-Systeme GmbH, 45309 Essen (DE)
(72) Erfinder: FALKENSTEIN, Martin, 44795 Bochum (DE)
(74) Vertreter: Bals, Rüdiger
(86) Internationale Anmeldenummer: PCT/EP2014/069217
(87) Internationale Veröffentlichungsnummer: WO 2015/036404

(56) Entgegenhaltungen:
- US-A- 4 745 794
- US-A- 5 604 297
- Anonymous: "Automatic CO2 Tester CO2MS-3V - Steinfurth", , 1. Januar 2012 (2012-01-01), Seiten 1-2, XP055161590, Gefunden im Internet: URL:https://web.archive.org/web/2012050308 0113/http:/www.steinfurth.de/page,co2-meas uring-instruments,automatic-co2-tester-co2 ms-3v,0,0,40,0,en.htm [gefunden am 2015-01-13]
- "Produktbroschüre STEINFURTH CO2 -TESTER CO2 MS", , 1. Januar 2012 (2012-01-01), Seiten 1-2, XP055161592, Gefunden im Internet: URL:https://web.archive.org/web/2015011309 3305/http:/www.steinfurth.de/files/CO2MS3V _e.pdf [gefunden am 2015-01-13]
- Johann Angres: "If it ain't broke... What is important in measuring CO2 content?", Brewer & Distiller International (www.ibd.org.uk), 1. November 2010 (2010-11-01), Seiten 1-2, XP055161594, Gefunden im Internet: URL:http://www.steinfurth.de/files/2010-11 -Brewer-Distiller-International-Steinfurth -What-is-important-in-measuring-CO2-conten t.pdf [gefunden am 2015-01-13]

## Beschreibung

Die vorliegende Erfindung bezieht sich auf eine Vorrichtung zur Ermittlung zumindest einer physikalischen, chemischen und/oder biologischen Eigenschaft eines innerhalb eines verschließbaren Behältnisses eingebrachten flüssigen Mediums. Des Weiteren betrifft die vorliegende Erfindung auch ein Verfahren zur Ermittlung zumindest einer physikalischen, chemischen und/oder biologischen Eigenschaft eines innerhalb eines verschließbaren Behältnisses eingebrachten flüssigen Mediums.

Grundlegend sind Verfahren insbesondere zur Untersuchung der Haltbarkeit von beispielsweise Lebensmitteln in Verpackungen, wie Kunststoffverpackungen, bekannt. Derartige Verfahren betreffen demzufolge vornehmlich Getränke oder Milchprodukte, in Form von Käse oder Joghurt, sowie Fleischwaren, Süßwaren, Gewürze und dergleichen. Über die Angabe eines Mindesthaltbarkeitsdatums des Lebensmittels, welche beispielsweise an dessen Verpackung angegeben wird, kann eine Aussage darüber getroffen werden, bis zu welchem Termin das entsprechende Lebensmittel bei sachgerechter Aufbewahrung auf jeden Fall ohne Einbußen im Geschmack und in der Qualität sowie ohne gesundheitliches Risiko zu konsumieren ist. Ein Mindesthaltbarkeitsdatum findet sich jedoch nicht nur auf den Verpackungen für entsprechende Lebensmittel, sondern ebenfalls auf Verpackungen für kosmetische Produkte, welche in der Regel nicht länger als ca. 2,5 Jahre haltbar sind. Da die Festlegung des Mindesthaltbarkeitsdatums im Ermessen des jeweiligen Produktherstellers ist, verwahren diese von den entsprechenden Herstellungsserien bzw. - chargen einzelne Proben, anhand welcher die Qualität der gesamten Charge bzw. Serie der hergestellten Produkte nachgewiesen werden kann. Eine derartige Stichprobenkontrolle der Serie bzw. Charge der Produkte mittels einzelner Produktproben ermöglicht dem Produkthersteller eine einfache Qualitätskontrolle des hergestellten Produktes. Wird bei der Produktqualitätskontrolle ein Produkt mit nicht hinreichender Qualität erkannt, so hat der Hersteller die Möglichkeit, die gesamte Produktionscharge zu sperren und eine Lieferung an die Konsumenten zu verhindern.

Insbesondere im Bereich der Getränkeindustrie sind der vorhandene Druck in der Flasche sowie das Material der Flasche selbst, welche beispielsweise aus Glas, Porzellan oder Kunststoff bestehen kann, maßgeblich für die Qualität und den Geschmack der Getränke selbst. Insbesondere beispielsweise auch der Kohlensäuregehalt bzw. der Kohlendioxidgehalt (CO₂-Gehalt) in den Getränken ist eine wichtige Einflussgröße hinsichtlich des Geschmackes und der Haltbarkeit des Getränkes bzw. des Lebensmittels. Um eine physikalische Eigenschaft, wie beispielsweise die Farbe, die Dichte oder die Viskosität insbesondere eines flüssigen oder fließfähigen Mediums, wie beispielsweise eines Getränkes, oder auch dessen chemische Eigenschaft, wie beispielsweise den Kohlendioxidgehalt und/oder auch dessen biologische Eigenschaft, wie beispielsweise ein Bakterienvorkommen, ermitteln zu können, bedarf es neben einer optimalen Probenvorbereitung - für die ausreichend viel Zeit benötigt wird - auch einer entsprechenden Messmethodik, welche reproduzierbare Messergebnisse liefert. Daneben sind auch sämtliche Verpackungseinflüsse auf die Qualität des flüssigen Mediums mit zu berücksichtigen. Insbesondere der kontinuierliche Trend zu immer neuen und auch immer günstigeren Verpackungsarten und Verpackungsmaterialien zum Verpacken der flüssigen Medien macht eine Ermittlung bzw. Messung der physikalischen, chemischen und/oder biologischen Eigenschaften des flüssigen Mediums ohne eine Berücksichtigung der jeweiligen Verpackungseinflüsse unvollkommen.

Die US 4 745 794 beschreibt ein Messgerät für die automatische Messung des CO₂-Gehaltes eines Getränkes, mit einer durch Ventile verschließbaren Messzelle samt integriertem Druck- und Temperaturaufnehmern, einer Auswerteeinheit sowie einer Bewegungseinrichtung für das Behältnis.

Eine Aufgabe der vorliegenden Erfindung ist es folglich, die zuvor beschriebenen Nachteile zumindest teilweise zu überwinden. Insbesondere ist es die Aufgabe der vorliegenden Erfindung ein Verfahren sowie eine Vorrichtung zur Ermittlung zumindest einer physikalischen, chemischen und/oder biologischen Eigenschaft eines flüssigen bzw. fließfähigen Mediums zur Verfügung zu stellen, welches schnell und zuverlässig durchführbar ist. Zusätzlich sind eine einfache Bedienung sowie eine wartungsarme Konstruktion wünschenswert. Ebenfalls soll eine hohe Prozesssicherheit und eine gute Effizienz bei dem erfindungsgemäßen Verfahren und der erfindungsgemäßen Vorrichtung vorgesehen sein. Ferner lässt sich die vorliegende Erfindung in einfacher Art und Weise in bereits bestehende Qualitätssicherungssysteme integrieren.

Die vorliegende Aufgabe wird durch eine erfindungsgemäße Vorrichtung zur Ermittlung zumindest einer physikalischen, chemischen und/oder biologischen Eigenschaft eines innerhalb eines verschließbaren Behältnisses eingebrachten flüssigen Mediums gemäß den Merkmalen des Patentanspruchs 1 gelöst. Ebenfalls wird zur Lösung der Aufgabe ein Verfahren mit den Merkmalen des vorliegenden Patentanspruchs 10 beansprucht. In den abhängigen Vorrichtungs- und Verfahrensansprüchen sind bevorzugte Weiterbildungen der Erfindung aufgeführt. Merkmale, die zu dem erfindungsgemäßen Verfahren offenbart werden, gelten auch für die erfindungsgemäße Vorrichtung und umgekehrt. Außerdem kann das erfindungsgemäße Verfahren auf der erfindungsgemäßen Vorrichtung durchgeführt werden.

Die erfindungsgemäße Vorrichtung zur Ermittlung zumindest einer physikalischen, chemischen und/oder biologischen Eigenschaft eines innerhalb eines verschließbaren Behältnisses eingebrachten flüssigen bzw. fließbaren Mediums weist zumindest eine Messkopfeinrichtung auf, welche wenigstens eine Druckmesseinheit zur Messung eines Mediumdruckes eines innerhalb des Behältnisses befindlichen gasförmigen Mediums und/oder des flüssigen Mediums und eine Temperaturmesseinheit zur Messung einer Mediumtemperatur des gasförmigen Mediums und/oder des flüssigen Mediums aufweist. Des Weiteren weist die erfindungsgemäße Vorrichtung eine Bewegungseinrichtung, zumindest zum Bewegen des Behältnisses, und eine Bestimmungseinrichtung, wenigstens zum Bestimmen der physikalischen, chemischen und/oder biologischen Eigenschaften des flüssigen Mediums aus wenigstens jeweils einem Wert hinsichtlich dem gemessenen Mediumdruck und der gemessenen Mediumtemperatur auf. Die Druckmesseinrichtung bzw. Druckmesseinheit der Messkopfeinrichtung selbst ist beispielsweise ein Drucksensor, welcher die physikalische Größe "Druck" und vornehmlich den Absolutdruck in eine elektrische Ausgangsgröße als Maß für den Druck umformen kann. Hinsichtlich des zu messenden Druckes werden die Drucksensoren beispielsweise unterschieden in Passivdrucksensoren, Relativdrucksensoren, Absolutdrucksensoren oder Differenzdrucksensoren usw. Die Temperaturmesseinheit weist zumindest einen Temperatursensor auf, der idealerweise am oder in der Messkopfeinrichtung angeordnet ist. Auch die gemessene Temperatur kann in eine elektrische Ausgangsgröße als Maß dafür umgeformt werden. Die erwähnte Bewegungseinrichtung selbst ist insbesondere eine Dreh- und/oder Schwenkbewegungseinrichtung, welche das Behältnis beispielsweise entlang einer definierten Bewegungsbahn mit einer definierten Bewegungsgeschwindigkeit bewegen kann. Demnach ist es denkbar, dass das Behältnis beispielsweise entlang einer Kreisbahn oder auch einer elliptischen Bahn, bewegt und hierbei insbesondere geschwenkt bzw. gekippt wird. Es ist zudem auch möglich, dass das Behältnis entlang einer geraden Bewegungsbahn auf und ab und/oder vor und zurück, das heißt in translatorischer Bewegungsrichtung bewegt und insbesondere geschüttelt wird. Des Weiteren ist es möglich, dass das Behältnis um einen definierten Drehpunkt herum gedreht bzw. geschwenkt wird, sodass das Behältnis zumindest zeitweise und wenigstens abschnittsweise um eine eigene Achse, welche vorteilhaft senkrecht zu einer Längsachse des Behältnisses verläuft, gedreht wird. Wird das Behältnis um einen definierten Drehpunkt bzw. um eine definierte Drehachse gedreht bzw. geschwenkt, so verändert sich nicht nur die Ausrichtung des Behältnisses selbst, das heißt, dass der Bodenbereich des Behältnisses zumindest zeitweise und wenigstens teilweise in vertikaler Richtung betrachtet oberhalb des oberen bzw. Einführbereiches des Behältnisses angeordnet ist. Vielmehr verursacht die Dreh- bzw. Schwenkbewegung auch ein Fließen des innerhalb des Behältnisses eingebrachten flüssigen Mediums, insbesondere in den Mündungs- bzw. Öffnungsbereich des Behältnisses, ausgehend vom Bodenbereich und zu diesem Bodenbereich wieder zurück. Demzufolge ist vorteilhaft das Behältnis mittels eines Verschlusselementes derart dicht verschlossen, dass bei einer Durchführung einer Dreh- bzw. einer Schwenkbewegung, sowie auch bei der Durchführung einer translatorisch ausgerichteten Schüttelbewegung ein Herausfließen bzw. Herausströmen des flüssigen Mediums sicher verhindert wird. Neben dem flüssigen Medium befindet sich in der Regel auch ein gasförmiges Medium, wie beispielsweise Luft oder auch ein Luft-Kohlendioxid-Gemisch, in dem verschlossenen Behältnis. Bei der Dreh- bzw. Schwenkbewegung oder auch der Schüttelbewegung des Behältnisses wird folglich das flüssige Medium mit dem gasförmigen Medium vermengt bzw. weiteres gasförmiges Medium tritt aus dem flüssigen Medium aus und vermengt sich mit dem bereits bestehenden gasförmigen Medium. Vorteilhaft findet die Vermengung der Medien solange statt, bis ein Phasengleichgewicht bzw. ein Gleichgewichtszustand zwischen dem gasförmigen Medium und dem flüssigen Medium innerhalb des insbesondere geschlossenen Behältnisses erreicht ist. Unter dem Phasengleichgewichtszustand wird insbesondere ein chemisches Gleichgewicht verstanden, bei welchem die Konzentration bzw. die Menge von Produkten und Edukten nicht mehr verändert wird. In einem chemischen Gleichgewicht sind die Geschwindigkeiten von Hin- und Rückreaktion gleich groß. So ist es denkbar, dass vor der Ermittlung der physikalischen, chemischen und/oder biologischen Eigenschaft des flüssigen Mediums, das heißt vor der Probenmessung, das Behältnis zusätzlich auf eine vordefinierte Temperatur gebracht wird, um einen Gleichgewichtszustand zu erreichen.

Bei dem genannten flüssigen Medium handelt es sich vorteilhaft um ein Lebensmittel, insbesondere ein Getränk, vornehmlich ein kohlenstoffdioxidhaltiges (CO₂-haltiges) Getränk.

Es ist des Weiteren denkbar, dass zumindest vor der Ermittlung der physikalischen, chemischen und/oder biologischen Eigenschaft des flüssigen Mediums wenigstens eine geometrische Eigenschaft des Behältnisses, in welchem das Medium angeordnet ist, messtechnisch, insbesondere rein optisch erfasst wird.

Vorteilhaft dient die erfindungsgemäße Vorrichtung demzufolge dazu, die Probenvorbereitung, das heißt die Durchmischung des gasförmigen mit dem flüssigen Medium, mit einem Messgerät zur Ermittlung einer physikalischen, chemischen und/oder biologischen Eigenschaft des flüssigen Mediums zu kombinieren. Dadurch kann die Anschaffung und auch der Betrieb eines zusätzlichen Gerätes erspart werden, wodurch nicht nur die Prozess- bzw. Taktzeit bei der Ermittlung der Eigenschaft des flüssigen Mediums reduziert wird, sondern zudem auch der Platzbedarf in beispielsweise einem entsprechenden Messlabor verringert wird. Des Weiteren können vorteilhaft mit der erfindungsgemäßen Vorrichtung und folglich durch die Ermittlung der Eigenschaften des flüssigen Mediums, welches sich innerhalb des verschließbaren Behältnisses befindet, auch die möglichen Verpackungseinflüsse auf das flüssige Medium bei der Ermittlung der Eigenschaft berücksichtigt werden. Vorteilhaft können des Weiteren unnötige Störfaktoren bei der Ermittlung der Eigenschaft des flüssigen Mediums, welche beispielsweise bei einem Umrüsten des Behältnisses von dem Probenvorbereitungsgerät auf das Messgerät entstehen können, durch das direkte Messen des flüssigen Mediums in der Verpackung mit nur einer erfindungsgemäßen Vorrichtung verhindert werden.

Im Rahmen der Erfindung ist es denkbar, dass die Vorrichtung derart ausgestaltet ist, dass eine Probenvorbereitung simultan zur Ermittlung einer physikalischen, chemischen und/oder biologischen Eigenschaft des flüssigen Mediums durchführbar ist. Hierfür sind elektrische Schleifkontakte zwischen einem stationären Basisteil bzw. Gestell der Vorrichtung und der Messkopfeinrichtung vorgesehen, um elektrische Meßsignale von der Messeinheit von der Messkopfeinrichtung, die durch die Bewegungseinrichtung bei der Probenvorbereitung mit bewegt wird, auf den stationären Basisteil zur weiteren Verarbeitung zu übertragen. In einer anderen Ausführungsform werden die elektrische Meßsignale der Messeinheit von der Messkopfeinrichtung über eine drahtlose Verbindung auf das stationäre Basisteil zur weiteren Verarbeitung übertragen. Als drahtlose Verbindung kann beispielsweise eine Funkverbindung, wie z. B. BlueTooth, NFC, ZigBee, WIMAX, WISA oder andere WLAN Verbindungen genutzt werden. Dafür ist sowohl in der Messkopfeinrichtung als auch in dem Basisteil ein entsprechendes Kommunikationsmodul für die Funkverbindung angeordnet, die untereinander die elektrischen Meßsignale oder weitere Daten austauschen können.

Die Bestimmungseinrichtung, welche vorteilhaft zur Bestimmung und Auswertung der Eigenschaft des flüssigen Mediums dient, wird vorzugsweise vollautomatisch oder auch teilautomatisch betrieben.

Des Weiteren ist es denkbar, dass die Messkopfeinrichtung einen Anstichdorn zum Durchdringen eines Bereiches einer Wandung des Behältnisses und folglich zum Einbringen der Temperaturmesseinheit und/oder der Druckmesseinheit in einen Innenbereich des Behältnisses aufweist. Vorteilhaft wird insbesondere ein Bereich eines Verschlusselementes durchdrungen, insbesondere wenn das Behältnis selbst aus einem Glas bzw. glasartigen Material besteht. Vorliegend kann das Verschlusselement als Bestandteil des Behältnisses selbst angesehen werden, welches neben dem Verschlusselement auch eine Behältniswandung bzw. einen Behältniskorpus aufweisen kann. Die Messkopfeinrichtung selbst ist vorteilhaft eine verfahrbare bzw. eine schwenkbare Einrichtung, welche insbesondere auf einer Öffnung des Aufnahmebehälters, der zum wenigstens abschnittsweisen Aufnehmen des Behältnisses dient, aufgebracht werden kann. Ferner ist es denkbar, dass die Messkopfeinrichtung mit der Bewegungseinrichtung mechanisch verbunden ist und von dieser mit dem Behältnis mit bewegt und/oder geschüttelt wird. Dabei kann die Messkopfeinrichtung mit einem Dichtungselement oder Dichtring von außen auf das Behältnis, insbesondere das Verschlusselement gepresst werden, um den Bereich um den Anstichdorn sicher und zuverlässig druckdicht abzudichten.

Vorteilhaft erstreckt sich die Temperaturmesseinheit zumindest bereichsweise entlang des Anstichdorns, sodass bei einem Einstechen des Anstichdorns in einen Bereich des Verschlusselementes des Behältnisses oder auch in einem Bereich der Wandung des Behältnisses nicht nur der Anstichdorn selbst bzw. ein Bereich des Anstichdorns selbst in einem Innenbereich des Behältnisses eingebracht wird, sondern gleichzeitig auch die Temperaturmesseinheit bzw. ein Abschnitt der Temperaturmesseinheit durch den Anstichdorn in den Innenbereich des Behältnisses eingebracht wird. So ist es zudem denkbar, dass nicht nur die Temperaturmesseinheit, sondern gleichzeitig auch eine Druckmesseinheit in den Innenbereich des Behältnisses eingebracht wird, wobei es auch möglich ist, dass die Druckmesseinheit selbst in dem Bereich der Messkopfeinrichtung verbleibt.

Der Temperatursensor, welcher beispielsweise ein elektrischer oder auch elektronischer Sensor ist, welcher ein elektrisches Signal als Maß für die Temperatur liefert, kann beispielsweise als Heißleiter oder als Kaltleiter oder auch als integrierter Halbleitertemperatursensor ausgestaltet sein. Es ist des Weiteren denkbar, dass insbesondere neben der Anordnung einer Temperaturmesseinheit bzw. eines Temperatursensors und der Anordnung einer Druckmesseinheit bzw. eines Drucksensors die erfindungsgemäße Vorrichtung oder auch die Messkopfeinrichtung selbst weitere Sensoren aufweist, wie beispielsweise einen optischen Sensor, einen Gewichtssensor, einen Feuchtigkeitssensor, einen Piezosensor, einen kapazitiven und/oder induktiven Sensor usw. Mithilfe der unterschiedlichen Sensoren ist es folglich denkbar, unterschiedliche Eigenschaften des flüssigen sowie auch eventuell des gasförmigen Mediums innerhalb des Behältnisses zu detektieren bzw. zu messen. Vorteilhaft ist der Anstichdorn fest mit einer Oberfläche der Messkopfeinrichtung verbunden, sodass bei einer Bewegung der Messkopfeinrichtung selbst auf das Behältnis bzw. zu dem Behältnis hin auch der Anstichdorn auf das Behältnis bzw. zu dem Behältnis hin bewegt wird. Folglich ist es möglich, dass der Anstichdorn ein spitzes distales Ende, insbesondere nadelförmiges Ende aufweist, welches zum Eindringen in den Innenbereich des Behältnisses erforderlich ist. Aufgrund des Durchdringens bzw. Durchstechens eines Bereiches des Behältnisses bzw. des Verschlusselementes des Behältnisses mittels des Anstichdorns wird insbesondere eine zerstörende Prüfung des Behältnis bzw. des Verschlusselementes realisiert.

Der Aufnahmebehälter bzw. die Aufnahmevorrichtung, welcher wenigstens zum abschnittsweisen Aufnehmen des Behältnisses dient und das Behältnis teilweise oder ganz bis zu dessen Öffnungs- bzw. Mündungsbereich aufnehmen kann, ist vorteilhaft zumindest teilweise optisch durchsichtig ausgestaltet oder weist zumindest ein optisch durchsichtiges Fenster auf. So ist es auch denkbar, dass der Aufnahmebehälter das Behältnis druckdicht aufnimmt und umschließt, wobei der Aufnahmebehälter vorteilhaft die konstruktive Ausgestaltung des Behältnisses aufweist. Das Behältnis zur Aufnahme des flüssigen Mediums ist beispielsweise eine Flasche, in welcher ein Getränk, wie beispielsweise ein Wasser oder ein limonadenhaltiges Getränk, abgefüllt ist. Als Verschlusselement dient beispielsweise ein Schraubverschluss, ein Korkverschluss oder auch ein Kronkorkenverschluss oder ein vergleichbarer Verschluss. Bei dem Behältnis zur Aufnahme des flüssigen Mediums kann es sich explizit um eine Glas- oder Kunststoffbehälter, insbesondere aus PET (Polyethylenterephthalat) handeln. Gerade Kunststoffbehälter sowie PET-Flaschen bzw. Behälter weisen das Problem auf, dass sie nicht über einen langen Zeitraum druckdicht und/oder CO₂-dicht sind.

Gemäß einer vorteilhaften Weiterentwicklung weist die Messkopfeinrichtung zumindest ein Dichtungselement zum abdichtenden Verschließen einer Öffnung des Aufnahmebehälters und/oder zum abdichtenden Verschließen einer Öffnung des Behältnisses und insbesondere des Verschlussbereiches auf. Demzufolge weist die Messkopfeinrichtung insbesondere einen Dichtring oder eine Dichtkante aus insbesondere einem kautschukhaltigen Dichtungsmaterial auf, welcher sich beim Anordnen der Messkopfeinrichtung an dem Behältnis und folglich an dem Aufnahmebehälter derart zwischen der Messkopfeinrichtung und der Öffnungskante des Aufnahmebehälters bzw. zwischen der Messkopfeinrichtung und einer Oberfläche des Verschlusselementes des Behältnisses anlegt, dass ein Austreten, insbesondere eines gasförmigen Mediums, verhindert werden kann.

Es ist des Weiteren denkbar, dass die erfindungsgemäße Vorrichtung wenigstens ein Spannelement zum Arretieren der Messkopfeinrichtung im Bereich einer Öffnung des Aufnahmebehälters an einer Oberfläche einer Außenseite des Aufnahmebehälters aufweist. Vorteilhaft wird mithilfe des Spannelementes ein Anordnen und/oder Arretieren der Messkopfeinrichtung auf einer Öffnung des Aufnahmebehälters und/oder einem oberen Bereich, insbesondere dem Mündungsbereich und vorzugsweise dem Bereich, an welchem das Verschlusselement angeordnet ist, des Behältnisses ermöglicht. Demnach dient das Spannelement vorteilhaft zum Arretieren der Messkopfeinrichtung auf der Öffnung des Aufnahmebehälters und folglich zum Einspannen des Behältnisses in dem Aufnahmebehälter, das heißt zwischen dem Aufnahmebehälter und der Messkopfeinrichtung selbst. Hierdurch kann auch ein Anpressdruck für das Dichtungselement zwischen Messkopfeinrichtung und dem Aufnahmebehälter bzw. zwischen der Messkopfeinrichtung und einer Oberfläche des Verschlusselementes des Behältnisses erzeugt werden.

Gemäß einer vorteilhaften Weiterentwicklung weist die Temperaturmesseinheit ein rohrförmiges Isolationselement aus einem thermische Energie isolierenden Material auf, durch dessen Innenraum hindurch sich ein thermische Energie leitendes bzw. leitfähiges Übertragungselement erstreckt. Demzufolge ist es denkbar, dass das Isolationselement insbesondere aus einem karbonhaltigen oder auch keramischen oder auch kunststoffhaltigen Material besteht, während das Übertragungselement selbst entweder ein fadenförmiges Element ist, welches beispielsweise aus einem leitfähigen Metall, wie Silber, besteht. Es wäre jedoch auch möglich, dass das Übertragungselement selbst in Form einer sich an der Innenseite des Isolationselements entlang erstreckenden Nut ausgebildet ist, welche mit einem thermische Energie leitfähigen Material beschichtet ist. Es ist des Weiteren denkbar, dass die erfindungsgemäße Vorrichtung eine Temperiereinheit zum Temperieren des Behältnisses und insbesondere des flüssigen Mediums aufweist, um vorteilhaft eine Gleichgewichtslage innerhalb des Behältnisses zu erreichen.

Gemäß einer bevorzugten Weiterentwicklung der erfindungsgemäßen Vorrichtung weist die erfindungsgemäße Vorrichtung und/oder die Bestimmungseinrichtung der erfindungsgemäßen Vorrichtung eine Auswerteeinheit zum Auswerten der gemessenen Messwerte, wie dem Mediumdruck und der Mediumtemperatur, insbesondere in Berücksichtigung vorgegebener Korrelationswerte, auf. Demzufolge ist es denkbar, dass hinsichtlich bestimmter Arten eines flüssigen Mediums, das heißt hinsichtlich bestimmter unterschiedlicher Getränkearten, beispielsweise in Form einer Datenbank für unterschiedliche Drücke und Temperaturen, entsprechende physikalische, chemische und/oder biologische Eigenschaftswerte des flüssigen Mediums hinterlegt wurden. Auf diese hinterlegten Daten kann folglich die Auswerteeinheit der Bestimmungseinrichtung zugreifen. Es ist des Weiteren denkbar, dass die Bestimmungseinrichtung neben der Auswerteeinheit auch eine Anzeigeneinheit in Form eines Bildschirmes aufweist, mittels welcher neben den unterschiedlichen gemessenen Werten des Druckes und der Temperatur des flüssigen Mediums bzw. des gasförmigen Mediums auch der ermittelte Eigenschaftswert hinsichtlich der physikalischen, chemischen und/oder biologischen Eigenschaft des flüssigen Mediums angezeigt werden kann. Es ist des Weiteren möglich, dass die Anzeigeeinheit auch gleichzeitig als Eingabeeinrichtung dient, indem diese beispielsweise in Form eines berührungssensitiven Bildschirms ausgestaltet ist (Touch-Screen). Die Eingabeeinheit selbst kann jedoch auch eine separate Einheit sein, welche beispielsweise über die Bestimmungseinrichtung mit der Anzeigeeinheit verbunden ist. Über die Eingabeeinheit ist es beispielsweise einer Person möglich der Bestimmungseinrichtung vorzugeben, welche Art des flüssigen Mediums, das heißt insbesondere welche Art des Getränkes, sich in der aktuellen Flasche bzw. in dem aktuellen Probebehältnis befindet. Die Eingabe der Art des flüssigen Mediums dient insbesondere der Bestimmungseinrichtung und vornehmlich der Auswerteeinheit dazu, den entsprechenden Eigenschaftswert aus der Datenbank in Berücksichtigung der ermittelten Temperatur und des ermittelten Druckes auslesen zu können. Es ist des Weiteren denkbar, dass zusätzlich zu der Eingabeeinheit oder auch alternativ zur Eingabeeinheit die Bestimmungseinrichtung eine Scaneinheit aufweist, mittels welcher beispielsweise ein Barcode, Strichcode, Farbcode, QR-Code usw., welcher sich an dem Behältnis oder an einer Verpackung der Charge des hergestellten Produktes befindet, eingelesen werden kann. Durch das Einlesen des Codes wird folglich der Bestimmungseinrichtung und insbesondere der Auswerteeinheit der Bestimmungseinrichtung mitgeteilt, welche Art des flüssigen Mediums sich innerhalb des Behältnisses befindet, welches in der Vorrichtung angeordnet wird. Des Weiteren ist es möglich, dass die Bestimmungseinrichtung eine Speichereinheit aufweist, in welcher die einzelnen gemessenen Werte, insbesondere hinsichtlich des Druckes und der Temperatur und/oder die Werte der dazu ermittelten Eigenschaften, insbesondere der physikalischen, chemischen und/oder biologischen Eigenschaften des flüssigen Mediums zumindest zeitweise und vorzugsweise langfristig gespeichert werden. Vorteilhaft ist ein Auslesen dieser Speichereinheit jederzeit möglich. Des Weiteren kann die Bestimmungseinrichtung auch eine Empfangseinheit und/oder eine Sendeeinheit bzw. eine kombinierte Empfangs-und Sendeeinheit aufweisen, welche zum einen die ermittelten bzw. gemessenen Daten hinsichtlich des Druckes und der Temperatur empfängt und den ermittelten Eigenschaftswert des flüssigen Mediums hinsichtlich der physikalischen, chemischen und/oder biologischen Eigenschaft des flüssigen Mediums an eine dritte separate Einheit, wie beispielsweise einen Server oder externen Computer usw., versenden kann. Demzufolge ist die erfindungsgemäße Vorrichtung einfach und kostengünstig in ein bestehendes Qualitätsmanagementsystem einzubauen und ist in der Lage, schnell und prozesssicher jederzeit abrufbare Daten hinsichtlich der Eigenschaften des flüssigen Mediums bzw. des hergestellten Getränkeprodukts zu ermitteln und an entsprechende externe Auswerteeinheiten zu übertragen.

Es ist des Weiteren möglich, dass der Aufnahmebehälter ein Behältereinsatzelement zur Höhenausrichtung und Zentrierung des Behältnisses innerhalb des Aufnahmebehälters aufweist. Das Behältereinsatzelement ist vorteilhaft ein separates Element, aufweisend zumindest teilweise ein starres, steifes bzw. unelastisches Material, wie beispielsweise einen Kunststoff. Des Weiteren ist das Behältereinsatzelement vorteilhaft jederzeit aus dem Aufnahmebehälter entnehmbar. Es ist somit denkbar, dass das Behältereinsatzelement entsprechend der konstruktiven Ausgestaltung der einzelnen Behältnisse ausgestaltet ist, sodass je nach konstruktiver Ausgestaltung des in den Aufnahmebehälter einzusetzenden Behältnisses ein entsprechendes Behältereinsatzelement ausgewählt werden kann. Das Behältereinsatzelement weist folglich zumindest eine Vertiefung und/oder eine Aussparung und/oder einen Vorsprung und/oder eine Wandung auf, mittels welcher das Behältnis innerhalb des Aufnahmebehälters angeordnet werden kann. Um zu überprüfen, ob das korrekte Behältereinsatzelement zur Höhenjustierung und Zentrierung eines bestimmten Behältnisses innerhalb des Aufnahmebehälters ausgewählt wurde, kann beispielsweise manuell eine in einem Prüflabor oder auch in einer Produktionslinie arbeitende Person, welche die Ermittlung einer Eigenschaft des flüssigen Mediums durchführt und überwacht, oder auch die Vorrichtung selbst automatisch mittels einer Höhenlehre oder einer Lichtschranke usw. die korrekte Höhenjustierung des Behältnisses im Aufnahmebehälter kontrollieren. Zur automatischen Kontrolle der Höhenjustierung und/oder auch der Zentrierung des Behältnisses im Aufnahmebehälter kann beispielsweise eine Lichtschranke eingesetzt werden. Die korrekte Höhenjustierung des Behältnisses innerhalb des Aufnahmebehälters ist insbesondere deswegen erforderlich, damit die Messkopfeinrichtung beim Anordnen auf dem Aufnahmebehälter zum einen nicht das Behältnis, insbesondere dessen Öffnungs- bzw. Mündungsbereich, zerstört bzw. beschädigt und zum anderen ein bewegungsfreies bzw. bewegungsarmes Anordnen des Behältnisses zwischen dem einen Bereich (Bodenbereich) des Aufnahmebehälters und der Messkopfeinrichtung gewährleistet werden kann, sodass bei einer Schüttelbewegung bzw. Dreh- oder Schwenkbewegung des Behältnisses dieses nicht innerhalb des Aufnahmebehälters verrutscht und gegebenenfalls die Messkopfeinrichtung und/oder den Anstichdorn beschädigt.

Gemäß einer vorteilhaften Weiterentwicklung der erfindungsgemäßen Vorrichtung weist diese eine Entnahmeeinheit zur Entnahme eines innerhalb des Behältnisses befindlichen gasförmigen Mediums auf, welche an einer Außenseite des Aufnahmebehälters bzw. des stationären Basisteils der erfindungsgemäßen Vorrichtung angeordnet ist und zumindest ein Leitungselement aufweist, welches sich von der Entnahmeeinheit bis zu dem Anstichdorn erstreckt. Zum gezielten Ansteuern und Ablassen ist ein Steuerventil im Bereich des Leitungselements vorgesehen, was dieses öffnen bzw. verschließen kann. Vorteilhaft dient die Entnahmeeinheit dazu, ein innerhalb des Behältnisses befindliches gasförmiges Medium, welches insbesondere aus einem Luft-Kohlendioxid-Gemisch besteht, zumindest zeitlich vor dem Beginn einer Ermittlung der Eigenschaft bzw. eines Eigenschaftswertes des flüssigen Mediums zu entnehmen. Die Entnahmeeinheit (Snift-Sammler) leitet das gasförmige Gemisch entweder in einen entsprechenden Aufnahmebehälter oder auch an die Umgebung weiter. Das Leitungselement der Entnahmeeinheit zum Leiten des gasförmigen Gemisches aus dem Innenbereich des Behältnisses nach außen, erstreckt sich vornehmlich von der Entnahmeeinheit über insbesondere den Anstichdorn in den Innenbereich des Behältnisses, sodass vorteilhaft kein zweiter Bereich des Behältnisses und insbesondere kein weiterer Wandungsbereich des Behältnisses durchdrungen werden muss, um eine Entnahme bzw. ein Abführen des gasförmigen Gemisches aus dem Behältnis zu ermöglichen.

Das erfindungsgemäße Verfahren zur Ermittlung zumindest einer physikalischen, chemischen und/oder biologischen Eigenschaft eines innerhalb eines verschließbaren Behältnisses eingebrachten flüssigen Mediums weist zumindest die folgenden Schritte auf:
- Kontinuierliches Messen eines Mediumdruckes eines innerhalb des Behältnisses befindlichen gasförmigen Mediums und/oder des flüssigen Mediums mittels einer Druckmesseinheit einer Messkopfeinrichtung sowie einer Mediumtemperatur des gasförmigen Mediums und/oder des flüssigen Mediums mittels einer Temperaturmesseinheit der Messkopfeinrichtung während einer Bewegung des Behältnisses,
- Detektieren einer Einstellung eines Phasengleichgewichtszustandes zwischen dem gasförmigen und dem flüssigen Medium innerhalb des Behältnisses,
- Übertragen der Messwerte mittels Schleifkontakten oder einem Kommunikationsmodul von der Messkopfeinrichtung an eine Bestimmungseinrichtung zum Bestimmen der physikalischen, chemischen und/oder biologischen Eigenschaft des flüssigen Mediums aus wenigstens jeweils einem Wert hinsichtlich dem gemessenen Mediumdruck und der gemessenen Mediumtemperatur.

Vorteilhaft wird folglich der Mediumdruck sowie auch die Mediumtemperatur kontinuierlich, das heißt in definierten Zeitabständen bzw. Zeitintervallen gemessen. Es ist folglich denkbar, dass der Mediumdruck und die Mediumtemperatur beispielsweise jede hundertstel oder zehntel Sekunde vorzugsweise jede Sekunde bzw. alle fünf oder alle zehn Sekunden über einen definierten Zeitraum gemessen werden, wobei der definierte Zeitraum sich vom Beginn des Messvorgangs bis zum Einstellen des Phasengleichgewichtszustandes bzw. des chemischen Gleichgewichtzustandes des gasförmigen Mediums und des flüssigen Mediums, welche sich innerhalb des Behältnisses befinden, erstreckt. Vorteilhaft werden nicht nur simultan der Druck und die Temperatur gemessen, sondern gleichzeitig auch das Behältnis entlang beispielsweise einer definierten Bewegungsbahn bewegt, wobei die Bewegungsbahn eine Kreisbahn oder auch eine elliptische Bahn sein kann. Wie zuvor beschrieben, ist es folglich denkbar, dass das Behältnis, welches vorteilhaft in einem

Aufnahmebehälter angeordnet ist und folglich zusammen mit dem Aufnahmebehälter bewegt wird, entweder entlang einer translatorischen Bahn geschüttelt oder auch um einen Drehpunkt bzw. eine Drehachse gedreht bzw. geschwenkt wird, sodass eine Vermischung des in dem Behältnis befindlichen gasförmigen Mediums mit dem in dem Behältnis befindlichen flüssigen Mediums stattfinden kann. Vorteilhaft rotiert das Behältnis gleichmäßig über Kopf, was vorteilhaft zu einem optimalen Phasengleichgewicht führt, welches wiederum die Grundlage darstellt für eine optimale Reproduzierbarkeit und Messgenauigkeit des Eigenschaftswertes des flüssigen Mediums. Vorteilhaft wird mit dem erfindungsgemäßen Verfahren das Verfahren zur Vorbereitung des flüssigen Mediums zur Ermittlung der Eigenschaft mit dem Verfahren zur Ermittlung der Eigenschaft selbst verbunden. Das bedeutet, dass das Verfahren zur Einstellung eines Phasengleichgewichtszustandes zwischen dem gasförmigen und dem flüssigen Medium innerhalb des Behältnisses mit dem Verfahren zur Messung des Mediumdruckes und der Mediumtemperatur und folglich mit dem Verfahren zur Ermittlung der Eigenschaft auf Basis der ermittelten Mediumtemperatur und des ermittelten Mediumdruckes vorteilhaft kombiniert ist. Hierdurch wird erreicht, dass die Probenvorbereitung direkt beim Erreichen des Phasengleichgewichts beendet werden kann, was nun direkt durch die erfindungsgemäße Vorrichtung messbar ist. Somit lässt sich die Zeit für die Probenvorbereitung auf ein absolutes zeitliches Minimum reduzieren. Außerdem wird durch die kontinuierliche Messung während der Bewegung des Behältnisses sichergestellt, dass sich das Phasengleichgewicht eingestellt hat. Im Phasengleichgewicht ändern sich die gemessenen Werte zeitlich nicht mehr oder schwanken nur unwesentlich im Bereich einer definierten Messwertspanne. Sobald die Messwerte sich -wie zuvor beschrieben - nicht mehr ändern, ist somit das optimale Phasengleichgewicht im Behältnis erreicht und die zuletzt erhaltenen Messwert können für die Ermittlung der physikalischen, chemischen und/oder biologischen Eigenschaft des flüssigen Mediums herangezogen werden.

Bei der Ermittlung der physikalischen, chemischen und/oder biologischen Eigenschaft des flüssigen Mediums wird vorteilhaft ein Kohlenstoffdioxid-Konzentrationswert des flüssigen Mediums ermittelt. Da insbesondere der Geschmack sowie auch die Haltbarkeit eines Getränkes innerhalb eines Behältnisses durch die Menge des gelösten Kohlenstoffdioxidgehaltes beeinflusst werden, dienen die erfindungsgemäße Vorrichtung sowie das erfindungsgemäße Verfahren vorteilhaft dafür, insbesondere den CO₂-Gehalt in beispielsweise Wein, Bier, Wasser oder Softgetränken zu ermitteln, um einen gleichmäßigen CO₂-Gehalt in den produzierten bzw. abgefüllten Getränken jeder Produktionscharge sicherstellen zu können. Die CO₂-Konzentration in dem flüssigen Medium kann hierbei insbesondere durch die Druck- und Temperaturmessung während des Einstellens eines Gleichgewichtszustandes der Gas- und Flüssigphase ermittelt werden. Insbesondere zur Ermittlung der Kohlenstoffdioxidkonzentration in dem flüssigen Medium wird im Hinblick auf das Henry-Gesetz der Partialdruck eines gasförmigen Mediums, welches über dem flüssigen Medium angeordnet ist, ermittelt, wobei dieser Partialdruck direkt proportional zu der Kohlendioxidkonzentration des flüssigen Mediums ist. Die Henry-Formel ist folglich die getränkespezifische Formel, in Abhängigkeit welcher insbesondere die Kohlenstoffdioxidkonzentration im flüssigen Medium ermittelt wird.

Weitere Maßnahmen und Vorteile der Erfindung ergeben sich aus den Ansprüchen, der nachfolgenden Beschreibung und den Zeichnungen. Ebenfalls gelten die offenbarten Merkmale aus der erfindungsgemäßen Vorrichtung auch für das erfindungsgemäße Verfahren und umgekehrt. In den Zeichnungen ist die Erfindung in einem schematischen Ausführungsbeispiel dargestellt. Merkmale aus den Ansprüchen und in der Beschreibung können jeweils einzeln für sich oder in beliebiger Kombination erfindungswesentlich sein.

Es zeigen:
- Fig. 1: eine schematische Ansicht einer Ausführungsform der erfindungsgemäßen Vorrichtung, und
- Fig. 2: ein schematisches Diagramm eines Ablaufs eines Verfahrens zur Ermittlung einer physikalischen, chemischen und/oder biologischen Eigenschaft eines flüssigen Mediums.

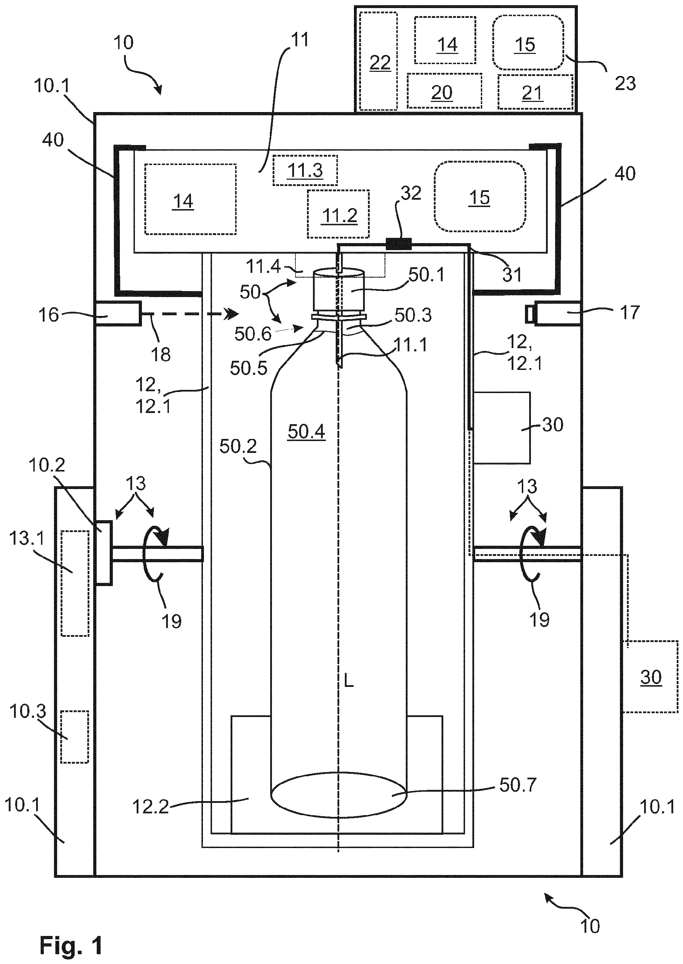

In der Figur 1 ist schematisch eine erfindungsgemäße Vorrichtung 10 zur Ermittlung zumindest einer physikalischen, chemischen und/oder biologischen Eigenschaft eines innerhalb eines verschließbaren Behältnisses 50 eingebrachten flüssigen bzw. fließfähigen Mediums 50.4, welches vorzugsweise ein Lebensmitteln und bevorzugt ein Getränk ist in Seitenansicht dargestellt. Die Vorrichtung 10 weist eine mechanische Aufnahmevorrichtung 12 bzw. einen mechanischen Aufnahmebehälter 12 zur Aufnahme und Anordnung des Behältnisses 50 an der Vorrichtung 10 auf. Die Behältnisse 50 sind vorteilhaft mit dem flüssigen Medium 50.4 befüllt, wobei die Befüllung des Mediums beispielsweise an einer nicht dargestellten Befüllstation stattfindet. Bei dem zu untersuchenden Behältnis 50 bzw. bei dem Behältnis 50, welches das zu untersuchende flüssige Medium 50.4 beinhaltet, handelt es sich insbesondere um einen Flüssigkeitsbehälter in Form einer Flasche 50.2, insbesondere Glas- oder Kunststoff-Flasche, wie z. B. PET-Flasche, welche mit einem Verschlusselement 50.1, wie beispielsweise einem Schraubverschlussdeckel, einem Kronkorkendeckel oder einem Korkstopfen oder einem vergleichbaren Deckel verschlossen ist. Ein Füllstand 50.5 des flüssigen Mediums 50.4 in dem Behältnis 50.2 ist ebenfalls schematisch angedeutet. Vorteilhaft ist das Behältnis 50.2 bis zu einem Halsbereich 50.6 bzw. bis in diesen Halsbereich 50.6 hinein mit dem flüssigen Medium 50.4 befüllt. Oberhalb des Füllstandes 50.5 befindet sich ein Kopfraum 50.3, der üblicherweise nicht mit dem flüssigen Medium 50.4, sondern mit einem gasförmigen Medium, welches üblicherweise Kohlenstoffdioxid (CO₂) haltig ist, gefüllt ist. Damit dieses Gas im Kopfraum 50.3 nicht entweichen kann, ist das Behältnis 50.2 mit dem Verschlusselement 50.1 verschlossen.

Wie ferner aus der Fig. 1 hervorgeht, ist das Behältnis 50 durch ein Anstechmittel 11.1 bzw. einen Anstichdorn 11.1 angestochen worden, welcher beispielsweise durch eine Wandung des Verschlusselementes 50.1 bis in das flüssige Medium 50.4 ragt. Im Rahmen der Erfindung ist es jedoch ausreichend, wenn der Anstichdorn 11.1 durch das Verschlusselement 50.1 bis in den Kopfraum 50.3 des Behältnisses 50.2 geführt wird bzw. hineinragt und folglich das flüssige Medium 50.4 bei einem aufrechten Stehen des Behältnisses 50.2 auf dessen Behältnisboden 50.7 nicht kontaktiert. Der Anstichdorn 11.1 ist vorteilhaft selbst ein Bestandteil des der Messkopfeinrichtung 11, welche oberhalb des Verschlusselementes 50.1 angeordnet ist. Dabei kann die Messkopfeinrichtung 11 auch zum Abdichten des Verschlusselementes 50.1 beim Anstechen dieses Verschlusselementes 50.1 mittels des Anstichdorn 11.1 und beispielsweise eines gestrichelt angedeuteten Dichtungselementes 11.4 dienen. Dieses angedeutete Dichtungselement 11.4 ist vorzugsweise zwischen der Messkopfeinrichtung 11 und dem Behältnis 50.2, insbesondere dem Verschlusselement 50.1 abdichtend eingeklemmt bzw. eingespannt. In der Messkopfeinrichtung 11, welche vornehmlich der erfindungsgemäßen Vorrichtung 10 zugeordnet ist, kann zumindest eine Messeinheit 11.2 und insbesondere ein Sensor 11.2 angeordnet sein. Bei dieser Messeinheit 11.2 kann es sich insbesondere um eine Temperaturmesseinheit bzw. einen Temperatursensor und/oder eine Druckmesseinheit bzw. einen Drucksensor handeln. Auch können mehrere Messeinheiten 11.2 in dem Messkopfeinrichtung 11 vorgesehen sein, die ebenfalls die Eigenschaften des flüssigen Mediums 50.4 durch den Anstichdorn 11.1 direkt messtechnisch erfassen.

Des Weiteren ist die Vorrichtung 10 mit einer Bewegungseinrichtung 13 und insbesondere einer Dreh- und oder Schwenkeinrichtung 13 bzw. einem Dreh- und Schwenkmechanismus 13 ausgestattet. Vorteilhaft ist die Bewegungseinrichtung 13 im Wesentlichen bügelartig 12.1 ausgestaltet und dient dazu das Behältnis 50 sicher über den Aufnahmebehälter 12 aufzunehmen, bzw. zu fixieren. Am unteren, distalen Ende des Aufnahmebehälters 12 kann ein Behältereinsatzelement 12.2 angeordnet sein, welches neben der Zentrierung und Höhenjustierung des Behältnisses 50 innerhalb des Aufnahmebehälters 50.2 und folglich in der Vorrichtung 10 ebenfalls zur formschlüssigen und/oder kraftschlüssigen Halterung des Behältnisses 50 dient. Vorteilhaft kann folglich mittels des Aufnahmebehälters 12 und insbesondere dessen Behältereinsatzelement 12.2 das Behältnis 50 geometrisch exakt, das heißt zentriert und höhenjustiert innerhalb der Vorrichtung 10 justiert bzw. angeordnet werden. Die Messkopfeinrichtung 11, welche vorteilhaft ebenfalls an der Bewegungseinrichtung 13 mit dem Bügel 12.1 des Aufnahmebehälters 12 verbunden ist und mit bewegt wird, kann zusätzlich über eine Auswerteeinheit 14 sowie eine Anzeigeeinheit 15 verfügen. Es ist jedoch auch denkbar, dass die Messkopfeinrichtung 11 mit einer Bestimmungseinrichtung 23 datenübertragungstechnisch z. B. durch Schleifkontakte 10.2 via Leitung bzw. Kommunikationsmodule 10.3, 11.3 via Funk bzw. drahtlos verbunden ist, wobei die Bestimmungseinrichtung 23 neben der Auswerteeinheit 14 und der Anzeigeeinheit 15 auch eine Speichereinheit 20 zum Speichern eingegebener und/oder ermittelter Daten und Werte, wie den gemessenen Druckwerten und Temperaturwerten, sowie eine Sende-und Empfangseinheit 21 zum Empfangen der gemessenen Werte (Druck/Temperatur) von beispielsweise der Messkopfeinrichtung 11 sowie zum Versenden von über eine Eingabeeinheit 22 eingegebener Werte, wie beispielsweise Daten über die Art oder Zusammensetzung des zu untersuchenden flüssigen Mediums, die Art des Behältnisses 50 (Glasmaterial, Kunststoffmaterial etc.) aufweist. Es ist zudem möglich, dass die Anzeigeeinheit 15 in ein berührungssensitiver Bildschirm (Touchscreen) ist, über welchen folglich auch Daten bzw. Werte eingegebene werden können, sodass die Anzeigeeinheit 15 auch gleichzeitig eine Eingabeeinheit 23 darstellt und auf eine separate Eingabeeinheit 22 verzichtet werden kann. Die Datenübertragung zwischen der Messkopfeinrichtung 11 und der Bestimmungseinrichtung 23 erfolgt kabelgebunden über die Schleifkontakte 10.2 oder kabellos über die entsprechenden Kommunikationsmodule 10.3, 11.3 beispielsweise per Bluetooth oder Wireless-LAN. Es ist auch denkbar, dass die Bestimmungseinrichtung 23 mit den entsprechenden Einheiten 14, 15, 20, 21, 22 in der Messkopfeinrichtung 11 integriert ist und folglich einen Bestandteil der Messkopfeinrichtung 11 darstellt. Vorteilhaft ist die Bestimmungseinrichtung 23 stationär an der Vorrichtung 10 bzw. des Basisteils 10.1 der Vorrichtung 10 angeordnet und bewegt sich folglich nicht, wie dagegen die Messkopfeinrichtung 11, mit dem Aufnahmebehälter 12 und dem darin angeordneten Behältnis 50 um einen definierten Drehpunkt bzw. eine definierte Drehachse , wie beispielsweise mit dem Bezugszeichen 19 gezeigt.

Es ist zudem denkbar, dass der Bügel 12.1 nicht nur an einem Aufnahmebehälter 12, welcher vornehmlich als zumindest teilweise geschlossenes Gehäuse, aufweisend eine Eingabeöffnung, insbesondere in Form eines Zylinders ausgestaltet ist, angeordnet ist, und diesen Aufnahmebehälter 12 zumindest bereichsweise umgibt. Vielmehr kann der Bügel 12.2 auch selbst als Aufnahmemittel dienen und folglich den Aufnahmebehälter 12 weitestgehend ersetzten, sodass das Behältereinsatzelement 12.2 vorteilhaft direkt an dem Bügel 12.1 und insbesondere an dessen unterem, distalen Ende angeordnet ist, um das Behältnis 50 zu positionieren, zu justieren und zu zentrieren. Es ist des Weiteren denkbar, dass innerhalb des Aufnahmebehälters 12 zum Beispiel eine zuvor beschriebene, jedoch hier nicht dargestellte, Temperiereinheit angeordnet ist, welche zur Temperierung des flüssigen Mediums 50.4 dient, um die Einstellung eines Phasengleichgewichtszustandes innerhalb des Behältnisses 50 beschleunigen zu können.

Des Weiteren kann innerhalb der Vorrichtung 10, insbesondere im Bereich des Kopfraumes 50.3 des Behältnisses 50 zumindest eine Lichtquelle 16, z. B. in Form eines Lasers, angeordnet sein, welcher einen Lichtstrahl 18 aussendet, der durch das Behältnis 50 stahlt. Insbesondere ist die Lichtquelle 16 derart ausgerichtet, dass die ausgesandten Lichtstrahlen bzw. elektromagnetischen Wellen im Wesentlichen senkrecht auf eine Längsachse L des Behältnisses 50 treffen. An einer der Lichtquelle 16 gegenüberliegenden Seite des Behältnisses 50 kann ein optischer Sensor 17 innerhalb der Vorrichtung 10 angeordnet sein, der den ausgesandten Lichtstrahl 18 messtechnisch erfasst. Auch kann an der Lichtquelle 16 selbst ein optischer Sensor 17 vorgesehen sein, der einen Teil des reflektierten Lichtstahles 18 der Lichtquelle 16 misst. Durch die vorgesehene Lichtquelle 16 sowie den optischen Sensor 17 lässt sich eine zerstörungsfreie, indirekte Probenmessung des Behältnisses 50 mit dem flüssigen Medium 50.4 vornehmen. Vorteilhaft ist der ausgesendete Lichtstrahl 18 nicht auf das für den Menschen sichtbare Licht beschränkt, sodass auch Lichtstrahlen 18 einer anderen Wellenlänge denkbar sind.

Mit dem Bezugszeichen 40 ist schematisch ein Spannelement dargestellt, welches sich von einer Außenseite des Aufnahmebehälters 12 bis zu einem Bereich der Messkopfeinrichtung 11 erstreckt und folglich die Messkopfeinrichtung 11 mit dem Aufnahmebehälter 12 vorzugsweise fest verbindet und hierbei auch eine Einspannkraft auf das angedeutete Dichtungselement 11.4 ausübt.

Insbesondere um eine optimale Probenvorbereitung und vornehmlich die Einstellung eines Phasengleichgewichtszustandes innerhalb des Behältnisses 50 zu erreichen, ist die Vorrichtung 10 mit der bereits erwähnten Bewegungseinrichtung 13 bzw. dem Dreh- und/oder Schwenkmechanismus 13 ausgestattet. Dieser wird durch einen elektromechanischen Antrieb 13.1 angetrieben, der beispielsweise durch einen E-Motor realisiert werden kann. Hierbei kann es sich um einen präzisen Schrittmotor handeln. Der Dreh- und oder Schwenkmechanismus 13 dreht dabei den Bügel 12.2 und/oder den Aufnahmebehälter 12 mit dem daran bzw. darin fixierten Behältnis 50 und der an dem Aufnahmebehälter 12 angeordneten Messkopfeinrichtung 11. Auch ist es denkbar, dass zumindest eine Lichtquelle 16 und/oder ein optischer Sensor 17 an der dem Aufnahmebehälter 12 angeordnet und nicht stationär mit der Vorrichtung 10 verbunden ist/sind, wie in der Figur 1 beispielhaft dargestellt.

Des Weiteren ist in der Fig. 1 eine Entnahmeeinheit 30 gezeigt, welche auch als "Snift-Sammler" bezeichnet wird. Die Entnahmeeinheit 30 ist vornehmlich in einem Bereich des Aufnahmebehälters 12 und/oder des Bügels 12.1 angeordnet und kann sich folglich mit diesen Bauteilen mitbewegen bzw. um die Drehachse 19 mitdrehen. Es ist jedoch auch denkbar, dass die Entnahmeeinheit 30 stationär innerhalb bzw. an der Vorrichtung 10 angeordnet ist, wie optional zum besseren Verständnis in Fig. 1 zusätzlich gestrichelt angedeutet ist, und lediglich über entsprechende Verbindungselemente mit dem Aufnahmebehälter 12 bzw. dem Bügel 12.1 verbunden ist. In beiden Ausgestaltungsformen führt ein Leitungselement 31 zum Leiten insbesondere eines gasförmigen Mediums von der Entnahmeeinheit 30 bis zu dem Anstichdorn 11.1, welcher insbesondere an dessen distalen E, welches sich bis in das Innere des Behältnisses 50 hinein erstreckt, spitz ausgestaltet ist. Zur gezielten Öffnen bzw. Schließen des Leitungselements 31 ist ein Steuerventil 32 im Bereich des Leitungselement 31 angeordnet. Über den Anstichdorn 11.1 kann nun mit Hilfe der Entnahmeeinheit 30 ein gasförmiges Medium, wie beispielsweise Luft oder auch ein Luft-Kohlendioxid-Gemisch insbesondere aus dem Kopfraum 50.3 des Behältnisses 50 abgeführt werden. Dieses gasförmige Gemisch kann dabei an einen Behälter oder auch an die Umgebung abgeleitet werden. Vorteilhaft wird das gasförmige Gemisch noch vor dem Beginn der Probenmessung und vorzugsweise auch vor Beginn einer Bewegung des Behältnisses 50 zur Durchmischung der Medien und Einstellung des Phasengleichgewichtszustandes innerhalb des Behältnisses 50 entnommen, um vorzugsweise insbesondere in dem Behältnis 50 befindliche Lust auslassen zu können.

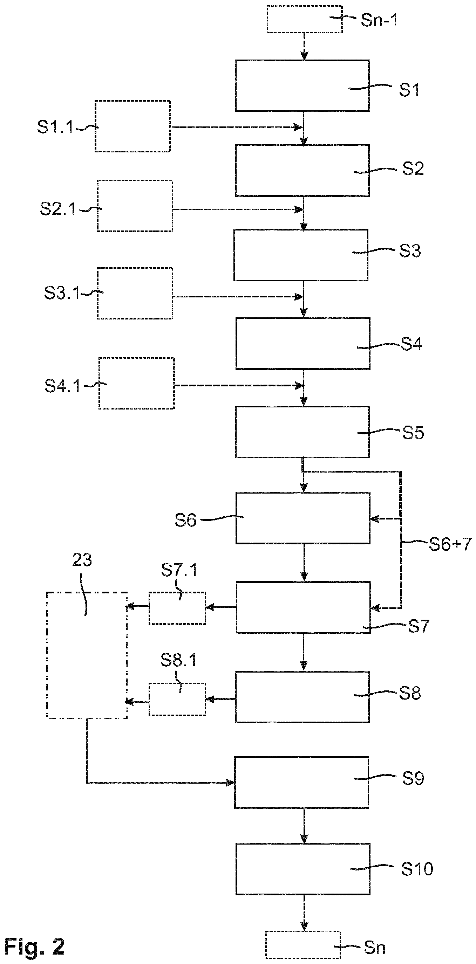

In der Fig. 2 ist schematisch ein möglicher Verfahrensablauf bei der Untersuchung der Probe und insbesondere für die Ermittlung einer physikalischen, chemischen und/oder biologischen Eigenschaft eines flüssigen bzw. fließfähigen Mediums aufgezeigt. Vornehmlich wird das Verfahren anhand der Ermittlung eines in dem flüssigen Medium 50.4 (vgl. Fig.1) befindlichen Kohlenstoffdioxidwertes beschrieben.

In einem ersten Schritt S1 wird das zu untersuchende Behältnis mit dem darin befindlichen zu untersuchenden flüssigen Medium an einen Bügel oder in einen Aufnahmebehälter, welcher vorteilhaft mit dem Bügel verbunden ist, angeordnet. Zur Zentrierung und Justierung, insbesondere der Höhe des Behältnisses innerhalb der Vorrichtung, wird ein Behältereinsatzelement basierend auf der konstruktiven Ausgestaltung des Behältnisses ausgewählt. Die Auswahl des Behältereinsatzelementes kann dabei manuell durch eine Person oder auch teilautomatisch bzw. vollautomatisch durch die Vorrichtung selbst erfolgen. Hierfür erkennt die Vorrichtung beispielsweise die Art und Ausgestaltung des Behältnisses über das Einlesen eines Codes mittels einer Scan-Einheit. Dazu führt vornehmlich eine Person die Scan-Einheit über den beispielsweise an dem Behältnis selbst oder auch an einer Großverpackung bzw. einem Gebinde zur Verpackung und Transport einer Charge von gleichen Behältnissen angebrachten Code, wie beispielsweis einem Barcode, QR-Code oder ähnlichem (teilautomatische Erkennung). Es ist jedoch auch denkbar, dass die Vorrichtung beispielsweise über eine Lasereinrichtung definierte Bereiche eines Behältnisses, welches in die Vorrichtung eingebracht werden soll, abtastet, um dadurch die Behältnisart ermitteln zu können (vollautomatische Erkennung). So ist es auch möglich, dass die Vorrichtung vollautomatisch einen Code des Behältnisses scannt oder die Lasereinheit nur mit Hilfe einer Person definierte Bereiche des Behältnisse abtasten kann.

Das ausgewählte Behältereinsatzelement wird vorzugsweise in einem Bodenbereich des Behältnisses angeordnet und vorteilhaft zusammen mit dem Behältnis in den Aufnahmebehälter eingebracht. Das vorteilhaft aus einen kunststoffartigen Material bestehende Behältereinsatzelement ist vornehmlich hitzebeständig, säurebeständig, einfach in dessen konstruktiver Ausgestaltung und kostengünstig in der Herstellung-. Pro Behältnisform gibt es jeweils ein Behältereinsatzelement, sodass jede erdenkliche Behältnisform innerhalb der Vorrichtung und insbesondere innerhalb eines Bereiches des Aufnahmebehälters angeordnet werden kann. Das Jeweilige Behältereinsatzelement ist insbesondere derart ausgestaltet, dass das Behältnis beim Anordnen in den Aufnahmebehälter zwischen 5 mm bis 7 mm über den Rand des Aufnahmebehälters hinausragt. Dadurch wird vorteilhaft ein dichtes Verschließen der Messkopfeinrichtung mit dem Aufnahmebehälter und insbesondere mit dem Mündungsbereich des Behältnisses selbst ermöglicht, ohne jedoch das Behältnis insbesondere in dessen Mündungsbereich bzw. im Bereich des Verschlusselementes zu beschädigen.

Fakultativ ist es möglich, dass in einem zweiten Schritt S2 der aus dem Aufnahmebehälter herausragende Bereich bzw. die Höhe des herausragenden Bereiches des Behältnisses manuell durch eine Person beispielsweise mittels einer mechanischen Höhenlehre oder automatisch durch die Vorrichtung selbst, beispielsweise mittels einer Lichtschranke nachgemessen wird, um gegebenenfalls ein zu gering oder auch zu weit aus dem Aufnahmebehälter herausragendes Behältnis erkennen und folglich ein anderes Behältereinsatzelement auswählen zu können.

Nachfolgend wird in einem dritten Schritt S3 die Messkopfeinrichtung mit angeordnetem Anstichdorn auf der Aufnahmeeinrichtung und insbesondere auf dem Behältnis platziert. Dabei ist es möglich, dass diese entlang einer Bewegungsbahn translatorisch nach unten gefahren wird, oder dass das Behältnis, vornehmlich gemeinsam mit dem Aufnahmebehälter und dem Bügel translatorisch nach oben zu der stationär angeordneten Messkopfeinrichtung gefahren wird. Vorzugsweise wird die Messkopfeinrichtung jedoch um einen definierten Drehpunkt bzw. um eine definierte Drehachse geschwenkt bzw. gekippt, und zwar soweit bis der Anstichdorn in einem mittleren Bereich des Verschlusselementes und vornehmlich im Bereich eines Mittelpunktes des Verschlusselementes angeordnet ist und das Verschlusselement folglich kontaktiert.

Fakultativ ist es möglich, dass in einem weiteren vierten Schritt S4 nun ein Teil eines Spannelementes, welches insbesondere an einem Außenumfang des Aufnahmebehälters angeordnet ist, mit einem Arretierungsbereich der Messkopfeinrichtung verbunden und insbesondere in diesen eingehakt wird. Nach dem Einhaken des Spannelementes wird dieses nun vorzugsweise manuell durch eine Person betätigt, wobei ein Hebelarm des Spannelementes umgelegt bzw. nach unten gedrückt wird. Dadurch zieht das Spannelement die Messkopfeinrichtung weiter nach unten in Richtung des Behältnisses, wodurch der Anstichdorn ebenfalls in Richtung des Behältnisses bewegt wird und die Wandung des Verschlusselementes durchdringt. Mittels eines im Bereich des Verschlusselementes an der Messkopfeinrichtung angebrachten Dichtungselementes wird vorteilhaft das Verschlusselement derart umgeben, dass ein Entweichen eines gasförmigen sowie auch eines flüssigen Mediums über die Einstichstelle im Verschlusselement nach außen in die Umgebung oder einen Bereich der Messkopfeinrichtung vermieden wird.

Weist die Vorrichtung kein Spannelement auf, wird beispielsweise mittels eines Motorelementes die Messkopfeinrichtung an dem Behältnis angeordnet und der Anstichdorn durch das Verschlusselement bewegt.

In einem weiteren Schritt S5 wird nun die Entnahmevorrichtung aktiviert, welche die in dem Behältnis befindliche Luft bzw. ein gasförmiges Gemisch, bestehend aus Luft und Kohlenstoffdioxid insbesondere aus dem Kopfraum entlässt.

In einem darauffolgenden sechsten Schritt S6 wird das Behältnis vorzugsweise gemeinsam mit der Messkopfeinrichtung und dem Aufnahmebehälter sowie dem Bügel bewegt und vorteilhaft geschüttelt bzw. um eine Drehachse geschwenkt. Beim Schwenken bzw. Drehen befindet sich der Bodenbereich zumindest zeitweise oberhalb des Halsbereiches des Behältnisses. Das Behältnis wird sozusagen über Kopf gedreht. Entweder jeweils in voller Umdrehung, nämlich 360°, wobei die Umdrehungsrichtung hierbei alternieren, das heißt wechseln kann. Es ist jedoch auch denkbar, dass das Behältnis jeweils nur um 180° in eine erste Richtung gedreht und danach in eine zweite Richtung, welche der ersten Richtung entgegengesetzt ist, um 180° in die Ausgangsposition zurück gedreht wird. Auch diese Drehrichtungen können sich abwechseln. Durch eine Bewegung des Behältnisses entweicht Kohlendioxyd aus dem flüssigen Medium.

In einem siebten Schritt S7 werden Werte hinsichtlich des Druckes und der Temperatur innerhalb der in dem Behältnis befindlichen Medien gemessen. Durch eine Schüttelbewegung oder auch die Drehbewegung ist es möglich, dass der Anstichdorn und folglich ein in dem Anstichdorn verlaufender Temperatursensor oder auch Drucksensor einmal das flüssige Medium und einmal das gasförmige Medium kontaktieren. Demzufolge werden, abhängig von dem Drehintervall oder dem Schüttelintervall und dem entsprechenden Messintervall, der Druck und/oder die Temperatur einmal des gasförmigen Mediums und dein anderes Mal des flüssigen Mediums gemessen bzw. ermittelt. So ist es denkbar, dass diese Messwerte der Medien abwechselnd vorliegen. In einem Schritt S7.1 werden die Messwerte vorteilhaft an eine Bestimmungseinrichtung 23 und insbesondere eine Auswerteeinheit, welche ein Bestandteil der Bestimmungseinrichtung 23 oder auch der Messkopfeinrichtung sein kann, gesandt und über eine Anzeigeeinheit angezeigt.

Vorzugweise wird ein Absolutdruck gemessen, der gemeinsam mit einem Relativdruck in der Anzeigeeinheit dargestellt wird. Die Druckanzeige kann in bar oder psi erfolgen, während die Temperaturanzeige in °Celsius oder auch in Fahrenheit erfolgen kann.

Vorteilhaft erfolgt das Messen von Temperatur und Druck kontinuierlich während des Bewegens des Behältnisses, wie durch den Schritt S6+7 verdeutlicht. Unter "kontinuierlich" wird hierbei das Messen in vornehmlich gleichmäßigen zeitlichen Abständen über eine definierte Zeitdauer verstanden, wobei die Messungen beispielsweise einmal oder mehrmals pro Sekunde oder einmal oder mehrmals pro Minute usw. erfolgen können. Die Zeitdauer erstreckt sich dabei vorteilhaft von beispielsweise einem Beginn des Bewegungsvorganges des Behältnisses bis zum Erreichen eines Gleichgewichtszustandes der in dem Behältnis befindlichen Medien.

Wird in einem achten Schritt S8 folglich ein Gleichgewichtszustand der innerhalb des Behältnisses befindlichen Medien ermittelt, das heißt wird weder eine Temperaturveränderung, noch eine Druckveränderung über zumindest zwei entweder aufeinanderfolgende Messwerte oder durch einen oder mehrere Messwerte beabstandete zwei Messwerte ermittelt, detektiert die Bestimmungseinrichtung 23 und insbesondere eine Auswerteeinheit nach einem Übersenden der Messwerte in einem Schritt S8.1 das Vorliegen eines Phasengleichgewichtszustandes. Die zu diesem Zeitpunkt des Vorliegens eines Phasengleichgewichtszustandes gemessenen Werte hinsichtlich des Druckes und der Temperatur werden dann als Basiswerte zur Bestimmung eines in dem flüssigen Medium befindlichen Kohlenstoffdioxidwert herangezogen.

Dazu liest die Auswerteeinheit in einem Schritt S9 aus den Eingangsgrößen, nämlich dem Druck und der Temperatur eine korrelierende Bezugsgröße, nämlich den Kohlenstoffdioxidgehalt, aus einer vorzugsweise in einer Speichereinheit der Bestimmungseinrichtung hinterlegten bzw. gespeicherten Datenbank bzw. Wertetabelle aus. Die korrelierenden Werte sind vor Beginn des Messverfahrens vornehmlich für unterschiedliche flüssige Medien hinterlegt wurden, sodass die Auswerteeinheit lediglich auf diese gespeicherten Werte nach Erfassung der Messwerte zurückgreifen kann.
In einem Schritt S10 wird dann der ausgelesene Kohlenstoffdioxidwert über eine Sende-und Empfangseinheit der Bestimmungseinrichtung an die Anzeigeeinheit übertragen und dort angezeigt.

Es ist zudem denkbar, dass auch ein Range, das heiß ein Wertebereich angezeigt wird, in welchem sich ein Kohlenstoffdioxid gemäß der vorliegenden Messwerte für das entsprechende flüssige Medium befinden müsste, damit die Haltbarkeit, die Qualität und auch der Geschmack des Mediums, sofern es sich um ein Lebensmittel handelt, gewährleistet ist. Vorteilhaft ist der ermittelte eigenschaftswert innerhalb dieses Wertebereiches aufgetragen bzw. abgebildet, sodass auf einfache Arte und Weise abgelesen werden kann, ob der Kohlenstoffdioxidgehalt beispielsweise in einem Grenzbereich liegt und bei der nächsten Produktionscharge mehr Kohlenstoffdioxid zugeführt werden muss, oder nicht.

Vor oder nach den Schritten S1, S2, S3 und/oder S4 ist es möglich, dass beispielsweise die Art des flüssigen Mediums, welches sich in dem Behältnis befindet, welches in die erfindungsgemäße Vorrichtung eingebracht wird, über eine Anzeigeeinrichtung durch eine Person, wie einen Labormitarbeiter oder einer an der Herstellung und/oder Abfüllung des Mediums beteiligten Person, eingegeben wird, wie durch die Schritte Sn-1, S1.1, S2.1, S3.1, S4.1 aufgezeigt.

Nach der Ermittlung einer Eigenschaft bzw. eines Eigenschaftswertes des flüssigen Mediums wird die Vorrichtung, wie insbesondere durch den Schritt Sn angedeutet, entweder manuell oder auch automatisch angehalten, wobei der Bewegungsvorgang sowie auch der Messvorgang gestoppt wird. Durch ein Lösen des Spannelementes und ein Öffnen bzw. Abheben der Messkopfeinrichtung kann dann das Behältnis wieder aus der Vorrichtung entfernt werden. Wegen des Vorliegens einer weitestgehend zerstörenden Prüfung, aufgrund beispielsweise des Durchstechens des Verschlusselementes mittels dem Anstichdorn und/oder aufgrund des Bewegens bzw. Schüttelns des Behältnisses und des Ausdiffundieren des gasförmigen Mediums aus dem flüssigen Medium, ist das Behältnis und auch die darin befindliche Flüssigkeit zu entsorgen.

### Bezugszeichenliste

- 10: Vorrichtung
- 10.1: stationäres Basisteil / Gestell von 10
- 10.2: Schleifkontakte
- 10.3: Kommunikationsmodul
- 11: Messkopfeinrichtung
- 11.1: Anstichdorn
- 11.2: Messeinheit / Druckmesseinheit / Temperaturmesseinheit
- 11.3: Kommunikationsmodul
- 11.4: Dichtungselement
- 12: Aufnahmebehälter
- 12.1: Bügel
- 12.2: Behältereinsatzelement
- 13: Bewegungseinrichtung / Dreh- und Schwenkbewegungseinrichtung, -mechanismus
- 13.1: Antrieb
- 14: Auswerteeinheit
- 15: Anzeigeeinheit
- 16: Lichtquelle, insbesondere Laser
- 17: Optischer Sensor für 16
- 18: Pfeil für Lichtstrahl
- 19: Pfeil für Drehrichtung
- 20: Speichereinheit
- 21: Sende- und/oder Empfangseinheit
- 22: Eingabeeinheit
- 23: Bestimmungseinrichtung

- 30: Entnahmeeinheit
- 31: Leitungselement
- 32: Steuerventil

- 40: Spannelement
- 50: Behältnis
- 50.1: Verschlusselement
- 50.2: Flasche
- 50.3: Kopfraum
- 50.4: flüssiges Medium
- 50.5: Füllstand
- 50.6: Halsbereich, -raum
- 50.7: Bodenbereich des Behältnisses 50

- L: Längsachse des Behältnisses 50

- Sn-1: möglicher Anfangsschritt: Eingabe von Daten
- S1: erster Schritt: Anordnen des Behältnisses in der Vorrichtung
- S1.1: möglicher Zwischenschritt: Eingabe von Daten
- S2: zweiter Schritt: Höhenkontrolle
- S2.1: möglicher Zwischenschritt: Eingabe von Daten

- S3: dritter Schritt: Anordnen der Messkopfeinrichtung
- S3.1: möglicher Zwischenschritt: Eingabe von Daten
- S4: vierter Schritt: Einspannen des Behältnisses
- S4.1: möglicher Zwischenschritt: Eingabe von Daten
- S5: fünfter Schritt: Aktivieren der Entnahmevorrichtung
- S6: sechster Schritt: Bewegen des Behältnisses
- S7: siebter Schritt: Messen von Druck und Temperatur
- S7.1: Zwischenschritt: Übertragen der Messwerte
- S8: achter Schritt: Detektieren eines Phasengleichgewichtszustandes
- S8.1: Zwischenschritt: Übertragen des Phasengleichgewichtszustandsdatums
- S9: neunter Schritt: Auslesen eines Eigenschaftswertes
- S10: zehnter Schritt: Anzeige des ermittelten Eigenschaftswertes
- Sn: letzter Schritt: Demontage des Behältnisses aus der Vorrichtung

## Patentansprüche

1. Vorrichtung (10) zur Ermittlung zumindest einer physikalischen, chemischen und/oder biologischen Eigenschaft eines innerhalb eines verschließbaren Behältnisses (50) eingebrachten flüssigen Mediums (50.4), aufweisend
- eine Messkopfeinrichtung (11) mit wenigstens einer Druckmesseinheit (11.2) zur Messung eines Mediumdruckes eines innerhalb des Behältnisses (50) befindlichen gasförmigen Mediums und/oder des flüssigen Mediums (50.4) und eine Temperaturmesseinheit (11.2) zur Messung einer Mediumtemperatur des gasförmigen Mediums und/oder des flüssigen Mediums (50.4),
- eine Bewegungseinrichtung (13) zumindest zum Bewegen des Behältnisses (50) und
- eine Bestimmungseinrichtung (23) wenigstens zum Bestimmen der physikalischen, chemischen und/oder biologischen Eigenschaft des flüssigen Mediums (50.4) aus wenigstens jeweils einem Wert hinsichtlich dem gemessenen Mediumdruck und der gemessenen Mediumtemperatur,
wobei zumindest Schleifkontakte (10.2) zwischen der Messkopfeinrichtung (11) und einem stationären Basisteil (10.1) der Vorrichtung (10) vorhanden sind, um über eine Leitung einen Datenaustausch zu ermöglichen, oder
dass jeweils ein Kommunikationsmodul (11.3, 10.3) an bzw. in der Messkopfeinrichtung (11) und dem Basisteil (10.1) der Vorrichtung (10) angeordnet sind, um einen drahtlosen Datenaustausch zu ermöglichen.

2. Vorrichtung (10) gemäß Anspruch 1,
**dadurch gekennzeichnet, dass**
die Messkopfeinrichtung (11) einen Anstichdorn (11.1) zum Durchdringen eines Bereiches einer Wandung des Behältnisses (50) und zum Einbringen der Temperaturmesseinheit (11.2) und/oder der Druckmesseinheit (11.2) in einen Innenbereich des Behältnisses (50) aufweist.

3. Vorrichtung (10) gemäß einem der vorangegangenen Ansprüche,
**dadurch gekennzeichnet, dass**
die Vorrichtung (10) einen Aufnahmebehälter (12) zum wenigstens abschnittsweisen Aufnehmen des Behältnisses (50) aufweist.

4. Vorrichtung (10) gemäß einem der vorangegangenen Ansprüche,
**dadurch gekennzeichnet, dass**
die Messkopfeinrichtung (11) zumindest ein Dichtungselement zum abdichtenden Verschließen einer Öffnung des Aufnahmebehälters (12) und/oder einer Öffnung des Behältnisses (50) aufweist.

5. Vorrichtung (10) gemäß einem der vorangegangenen Ansprüche,
**dadurch gekennzeichnet, dass**
wenigstens ein Spannelement (40) zum Arretieren der Messkopfeinrichtung (11) im Bereich einer Öffnung des Aufnahmebehälters (12) an einer Oberfläche einer Außenseite des Aufnahmebehälters (12) angeordnet ist.

6. Vorrichtung (10) gemäß einem der vorangegangenen Ansprüche,
**dadurch gekennzeichnet, dass**
die Temperaturmesseinheit (11.2) ein rohrförmiges Isolationselement aus einem thermische Energie isolierenden Material aufweist, durch dessen Innenraum sich ein thermische Energie leitfähiges Übertragungselement erstreckt.

7. Vorrichtung gemäß einem der vorangegangenen Ansprüche,
**dadurch gekennzeichnet, dass**
die Bestimmungseinrichtung (23) eine Auswerteeinheit (14) zum Auswerten der gemessenen Messwerte, wie dem Mediumdruck und der Mediumtemperatur, in Berücksichtigung vorgegebener Korrelationswerte aufweist.

8. Vorrichtung (10) gemäß einem der vorangegangenen Ansprüche,
**dadurch gekennzeichnet, dass**
die Vorrichtung (10) ein innerhalb des Aufnahmebehälters (12) anordenbares Behältereinsatzelement (12.2) zur Höhenausrichtung und Zentrierung des Behältnisses (50) aufweist.

9. Vorrichtung (10) gemäß einem der vorangegangenen Ansprüche,
**dadurch gekennzeichnet, dass**
eine Entnahmeeinheit (30) zur Entnahme eines innerhalb des Behältnisses (50) befindlichen gasförmigen Mediums in einem Bereich einer Außenseite des Aufnahmebehälters angeordnet ist und zumindest ein Leitungselement (31) der Entnahmeeinheit (50) sich von der Entnahmeeinheit (50) in Richtung des Anstichdorns (11.1) erstreckt.

10. Verfahren zur Ermittlung zumindest einer physikalischen, chemischen und/oder biologischen Eigenschaft eines innerhalb eines verschließbaren Behältnisses (50) eingebrachten flüssigen Mediums (50.4), aufweisend die Schritte:
- Kontinuierliches Messen eines Mediumdruckes eines innerhalb des Behältnisses (50) befindlichen gasförmigen Mediums und/oder des flüssigen Mediums (50.4) mittels einer Druckmesseinheit (11.2) einer Messkopfeinrichtung (11) sowie einer Mediumtemperatur des gasförmigen Mediums und/oder des flüssigen Mediums (50.4) mittels einer Temperaturmesseinheit (11.2) der Messkopfeinrichtung (11) während einer Bewegung des Behältnisses (50),
- Detektieren einer Einstellung eines Phasengleichgewichtszustandes zwischen dem gasförmigen Medium und dem flüssigen Medium (50.4) innerhalb des Behältnisses,
- Übertragen der Messwerte mittels Schleifkontakte (10.2) oder einem Kommunikationsmodul (11.3, 10.3) von der Messkopfeinrichtung (11) an eine Bestimmungseinrichtung (23) zum Bestimmen der physikalischen, chemischen und/oder biologischen Eigenschaft des flüssigen Mediums (50.4) aus wenigstens jeweils einem Wert hinsichtlich dem gemessenen Mediumdruck und der gemessenen Mediumtemperatur.

11. Verfahren gemäß dem vorangegangenen Anspruch 10,
**dadurch gekennzeichnet, dass**
wenigstens das Behältnis (50) mittels einer Bewegungseinrichtung (13) zumindest während der Dauer eines Messvorganges zum Messen des Mediumdruckes und der Mediumtemperatur entlang einer definierten Bewegungsbahn bewegt wird.

12. Verfahren gemäß einem der vorangegangenen Ansprüche 10 oder 11,
**dadurch gekennzeichnet, dass**
bei der Ermittlung der physikalischen, chemischen und/oder biologischen Eigenschaft des flüssigen Mediums (50.4) ein Kohlenstoffdioxid-Konzentrationswert des flüssigen Mediums ermittelt wird.

## Claims

1. Device (10) for determining at last one physical, chemical and/or biological characteristic of a liquid medium (50.4) introduced into a sealable container (50) comprising
- a measuring head system (11) with at least one pressure measuring unit (11.2) for measuring a medium pressure of a gaseous medium and/or the liquid medium (50.4) present in the container (50) and a temperature measuring unit (11.2) for measuring a medium temperature of the gaseous medium and/or the liquid medium (50.4),
- a movement device (13) at least for moving the container (50) and
- a determination system (23) at least for determining the physical, chemical and/or biological characteristic of the liquid medium (50.4) from at least one value relating to the measured medium pressure and the measured medium temperature in each case,
wherein slide contacts (10.2) are present between the measuring head system (11) and a stationary base section (10.1) of the device (10) in order to allow data exchange via a lead or a communication module (11.3, 10.3) is arranged in or on both the measuring head system (11) and the base section (10.1) of the device (10) in order to enable wireless data exchange to take place.

2. Device (10) in accordance with claim 1,
**characterised in that**
the measuring head system (11) has a spike (11.1) for piercing an area of a wall of the container (50) and to introduce the temperature measuring unit (11.2) and/or the pressure measuring unit (11.2) into an interior area of the container (50).

3. Device (10) according to any of the preceding claims,
**characterised in that**
the device (10) has a receiving container (12) for receiving the container (50) at least in sections.

4. Device (10) according to any one of the preceding claims,
**characterised in that**
the measuring head system (11) has at least one sealing element for the sealed closing of an opening of the receiving container (12) and/or an opening of the container (50).

5. Device (10) according to any one of the preceding claims,
**characterised in that**
at least one tension element (40) for locking the measuring head system (11) in an area of an opening of the receiving container (12) is arranged on a surface of an outer side of the receiving container (12).

6. Device (10) according to any one of the preceding claims,
**characterised in that**
the temperature measuring unit (11.2) has a tubular insulation element made of a thermal energy-insulating material, through the interior of which a thermal energy conducting transmission element extends.

7. Device according to any one of the preceding claims,
**characterised in that**
the determination system (23) has an evaluation unit (14) for evaluating the measured values such as the medium pressure and the medium temperature, taking into consideration predefined correlation values.

8. Device (10) according to any one of the preceding claims,
**characterised in that**
for adjusting the height and centring the container (50) the device (10) has a container insert element (12.2) which can be arranged in the receiving container (12).

9. Device (10) according to any one of the preceding claims,
**characterised in that**
an extraction unit (30) for extracting a gaseous medium present in the container (50) is arranged in an area of an outer side of the receiving container and at least one conduit element (31) of the extraction unit (30) extends from the extraction unit (30) in the direction of the spike (11.1).

10. Method of determining at least one physical, chemical and/or biological characteristic of a liquid medium (50.4) introduced into a sealable container (50) comprising at least the following stages:
- continuous measuring of a medium pressure of a gaseous medium present in the container (50) and/or of the liquid medium (50.4) by way of a pressure measuring unit (11.2) of a measuring head system (11) as well as of a medium temperature of the gaseous medium and/or the liquid medium (50.4) by way of a temperature measuring unit (11.2) of the measuring head system (11) during a movement of the container (50),
- detection of the establishment of a state of phase equilibrium between the gaseous and the liquid medium (50.4) in the container,
- transferring of the measurements from a measuring head system (11) to a determination system (23) by means of slide contacts (10.2) or a communication module (11.3, 10.3) in order to determine the physical, chemical and/or biological characteristic of the liquid medium (50.4) from at least one value, in each case relating to the measured medium pressure and the measured medium temperature.

11. Method according to the preceding claim 10,
**characterised in that**
the container (50) is moved along a defined movement path by means of a movement system (13) at least for the duration of a measuring procedure for measuring the medium pressure and the medium temperature.

12. Method according to any one of the preceding claims 10 or 11,
**characterised in that**
in the determination the physical, chemical and/or biological characteristic of the liquid medium (50.4) a carbon dioxide concentration value of the liquid medium is determined.

## Revendications

1. Dispositif (10) affecté à la définition d'au moins une propriété physique, chimique et/ou biologique d'un milieu fluide (50.4) logé à l'intérieur d'un réceptacle obturable (50), comprenant
- un appareil (11) à tête de mesure, présentant au moins une unité (11.2) de mesure de pressions qui est affectée à la mesure d'une pression d'un milieu gazeux situé à l'intérieur dudit réceptacle (50), et/ou dudit milieu fluide (50.4), et une unité (11.2) de mesure de températures qui est dédiée à la mesure d'une température dudit milieu gazeux et/ou dudit milieu fluide (50.4),
- un appareil (13) de mise en mouvement, au moins conçu pour mouvoir ledit réceptacle (50), et
- un appareil de détermination (23) au moins conçu pour déterminer la propriété physique, chimique et/ou biologique dudit milieu fluide (50.4) sur la base d'au moins une valeur respective se rapportant à la pression mesurée des milieux et à la température mesurée desdits milieux,
avec présence minimale de contacts frotteurs (10.2), entre l'appareil (11) à tête de mesure et une partie d'embase fixe (10.1) du dispositif (10), afin de permettre un échange de données par l'intermédiaire d'un conducteur, ou
d'un module respectif de communication (11.3, 10.3) respectivement placé sur, ou dans ledit appareil (11) à tête de mesure et ladite partie d'embase (10.1) dudit dispositif (10), afin de permettre un échange de données sans câble.

2. Dispositif (10) selon la revendication 1,
**caractérisé par le fait que**
l'appareil (11) à tête de mesure comporte une broche perforatrice (11.1) destinée à traverser une région d'une paroi du réceptacle (50), et à introduire l'unité (11.2) de mesure de températures et/ou l'unité (11.2) de mesure de pressions dans une région intérieure dudit réceptacle (50).

3. Dispositif (10) selon l'une des revendications précédentes,
**caractérisé par le fait que**
ledit dispositif (10) comporte un conteneur de réception (12) destiné à recevoir le réceptacle (50), au moins par zones.

4. Dispositif (10) selon l'une des revendications précédentes,
**caractérisé par le fait que**
l'appareil (11) à tête de mesure est pourvu d'au moins un élément d'étanchement conçu pour obturer, de manière étanche, une ouverture du conteneur de réception (12) et/ou une ouverture du réceptacle (50).

5. Dispositif (10) selon l'une des revendications précédentes,
**caractérisé par le fait**
**qu'**au moins un élément de serrage (40) est implanté sur une surface d'une face extérieure du conteneur de réception (12), en vue d'arrêter l'appareil (11) à tête de mesure dans la région d'une ouverture dudit conteneur de réception (12).

6. Dispositif (10) selon l'une des revendications précédentes,
**caractérisé par le fait que**
l'unité (11.2) de mesure de températures présente un élément tubulaire d'isolation en un matériau isolant de l'énergie thermique, à travers l'espace interne duquel s'étend un élément de transmission apte à conduire l'énergie thermique.

7. Dispositif (10) selon l'une des revendications précédentes,
**caractérisé par le fait que**
l'appareil de détermination (23) comporte une unité d'évaluation (14) destinée à évaluer les valeurs mesurées telles que la pression mesurée des milieux et la température mesurée desdits milieux, avec prise en compte de valeurs de corrélation préétablies.

8. Dispositif (10) selon l'une des revendications précédentes,
**caractérisé par le fait que**
ledit dispositif (10) comporte un élément (12.2) d'insertion du réceptacle, pouvant être placé à l'intérieur du conteneur de réception (12) en vue de l'orientation en hauteur et du centrage dudit réceptacle (50).

9. Dispositif (10) selon l'une des revendications précédentes,
**caractérisé par le fait**
**qu'**une unité de soutirage (30), conçue pour prélever un milieu gazeux situé à l'intérieur du réceptacle (50), est implantée dans une région d'une face extérieure du conteneur de réception, au moins un élément de conduit (31) de ladite unité de soutirage (30) s'étendant en direction de la broche perforatrice (11.1) à partir de ladite unité de soutirage (30).

10. Procédé de définition d'au moins une propriété physique, chimique et/ou biologique d'un milieu fluide (50.4) logé à l'intérieur d'un réceptacle obturable (50), incluant les étapes consistant à :
- mesurer en continu, lors d'un mouvement dudit réceptacle (50), une pression d'un milieu gazeux situé à l'intérieur dudit réceptacle (50), et/ou dudit milieu fluide (50.4), au moyen d'une unité (11.2) de mesure de pressions équipant un appareil (11) à tête de mesure, ainsi qu'une température dudit milieu gazeux et/ou dudit milieu fluide (50.4), au moyen d'une unité (11.2) de mesure de températures équipant ledit appareil (11) à tête de mesure,
- détecter un réglage d'un état d'équilibre de phases entre ledit milieu gazeux et ledit milieu fluide (50.4), à l'intérieur dudit réceptacle,
- transmettre les valeurs mesurées à partir dudit appareil (11) à tête de mesure, au moyen de contacts frotteurs (10.2) ou d'un module de communication (11.3, 10.3), à un appareil de détermination (23) conçu pour déterminer la propriété physique, chimique et/ou biologique dudit milieu fluide (50.4) sur la base d'au moins une valeur respective se rapportant à la pression mesurée des milieux et à la température mesurée desdits milieux.

11. Procédé selon la revendication 10 précédente,
**caractérisé par le fait**
**qu'**au moins le réceptacle (50) est mû le long d'une trajectoire de mouvement bien définie, au moyen d'un appareil (13) de mise en mouvement, au moins pendant la durée d'un processus de mesure en vue de mesurer la pression des milieux et la température desdits milieux.

12. Procédé selon l'une des revendications 10 ou 11 précédentes,
**caractérisé par le fait**
**qu'**une valeur de concentration en dioxyde de carbone du milieu fluide est définie au cours de la définition de la propriété physique, chimique et/ou biologique dudit milieu fluide (50.4).
